# EUROPÄISCHE PATENTSCHRIFT

(11) **EP 1 161 252 B1**
(45) Veröffentlichungstag und Bekanntmachung des Hinweises auf die Patenterteilung: **23.07.2003**
(21) Anmeldenummer: 00902530.5
(22) Anmeldetag: 17.02.2000
(51) Int. Cl.: A61K 35/78, A61P 25/20

(54) **VERWENDUNG VON EXTRAKTEN AUS GINKGO-BILOBA-BLÄTTERN ZUR BEHANDLUNG VON SCHLAFSTÖRUNGEN**
USE OF EXTRACTS FROM GINKGO BILOBA LEAVES FOR TREATING SLEEP DISORDERS
UTILISATION D'EXTRAITS DE FEUILLES DE GINKGO BILOBA POUR LE TRAITEMENT DE TROUBLES DU SOMMEIL

(30) Priorität: 16.03.1999 CH 47999
(43) Veröffentlichungstag der Anmeldung: 12.12.2001
(73) Patentinhaber: MEDICHEMIE AG, 4002 Basel (CH)
(72) Erfinder: WIDAUER, Josef, Olav, CH-4123 Allschwil (CH); HOLSBOER-TRÄCHSLER, Edith, 4123 Allschwil (CH); HATZINGER, Martin, 4125 Riehen (CH)
(74) Vertreter: Lauer, Joachim
(86) Internationale Anmeldenummer: CH0000087
(87) Internationale Veröffentlichungsnummer: WO00054791

(56) Entgegenhaltungen:
- DE-A- 4 110 620
- PATENT ABSTRACTS OF JAPAN vol. 1997, no. 05, 30. Mai 1997 (1997-05-30) & JP 09 023850 A (MORITANI KENKO SHOKUHIN KK), 28. Januar 1997 (1997-01-28) in der Anmeldung erwähnt
- PATENT ABSTRACTS OF JAPAN vol. 1997, no. 05, 30. Mai 1997 (1997-05-30) & JP 09 023849 A (MORITANI KENKO SHOKUHIN KK), 28. Januar 1997 (1997-01-28)
- PATENT ABSTRACTS OF JAPAN vol. 1997, no. 05, 30. Mai 1997 (1997-05-30) & JP 09 023851 A (MORITANI KENKO SHOKUHIN KK), 28. Januar 1997 (1997-01-28)

## Beschreibung

### TECHNISCHES GEBIET

Die vorliegende Erfindung bezieht sich auf das Gebiet der Mittel zur Behandlung von Schlafstörungen. Sie betrifft insbesondere ein Mittel zur Behandlung oder Zusatzbehandlung von Schlafstörungen, wie sie bei Patienten mit Depressionen oder im zunehmenden Alter zu beobachten sind.

### STAND DER TECHNIK

Extrakte der Blätter von Ginkgo Biloba, einem aus China stammenden Zierbaum, werden von alters her für diverse medizinische Anwendungen eingesetzt. Neben den alternativmedizinischen und homöopatischen Verwendungen sind im klassisch medizinischen Bereich unter anderem folgende Indikationen von Ginkgo biloba Extrakten bekannt: Durchblutungsförderung durch Verminderung der Plasmaviskosität und der Thrombocytenaggregation sowie Erhöhung der Kapillarpermeabilität, Förderung der Kompensation von Gleichgewichtsstörungen, Förderung der Durchblutung, insbesondere der Mikrozirkulation, Steigerung der Hypoxietoleranz, insbesondere des Himgewebes, sowie Verminderung des Retinaödems von Netzhautläsionen, und Steigerung der Gedächtnisleistung und des Lernvermögens.

Daneben ist aus der DE 41 10 620 (Deinlein-Kalb, H.) die Verwendung eines Extraktes aus Ginkgo Biloba Blättern als cerebrales Analeptikum nämlich zur Herstellung eines Medikamentes gegen die Apnoe, insbesondere die Schlafapnoe, bekannt. Die Schlafapnoe ist ein episodisch auftretendes Ausbleiben der Atmung während des Schlafes während mehr als 10 sec, was den Schlaf stört. Dennoch handelt es sich bei der Schlafapnoe definitionsgemäss nicht um eine Schlafstörung, sondern um eine Atemstörung, die bedingt wird durch eine Störung der zentralnervösen Regelung der Atemmuskulatur. Der Extrakt wirkt also hier als Analeptikum für die Regelmechanismen der Atemmuskulatur und verhindert wirksam die Apnoe während des Schlafens. Davon nochmals zu unterscheiden sind Atemaussetzer, die beim Schnarchen durch einen mechanischen Verschleiss der Atmungswege durch das zurückfallende Gaumensegel verursacht werden. Auch hierbei handelt es sich in der Fachsprache nicht um Schlafstörungen.

Ausserdem ist aus der DE 43 17 868 (Häcker, R. und Mattern, C.) die Verwendung eines Extraktes aus Ginkgo Biloba Blättern zusammen mit einem Extrakt aus Rhizoma zingiberis (Ingwerpulver) als Anxiolytikum, nämlich zur Herstellung eines Medikamentes gegen Angstzustände und zur Beruhigung bekannt. Das entsprechende Medikament ist für Patienten mit sogenanntem psychovegetativen Erschöpfungs-Syndrom verwendbar und führt zu einer Beruhigung der glatten Muskulatur und der allgemeinen Reizbarkeit unter einer gleichzeitigen Erhöhung der Vigilanz und der Spannkarft.

Aus den Patent Abstracts of Japan vol. 1997, no. 05 (1997-05-30) & JP-A-09 023849 - ...51 sind Mischungen aus pflanzlichen Extrakten enthaltend Ginkgo biloba zur Gewährleistung von ruhigem und gesundem Schlaf bekannt. Auch JP 6340539 beschreibt eine Mischung aus Ginkgo biloba Extrakt mit Docosahexaenoic acid zur Verbesserung seniler Dementia, wobei unter anderem günstige Auswirkungen auf den Schlaf der getesteten Personen festgestellt wurden.

### DARSTELLUNG DER ERFINDUNG

Gegenstand der Erfindung ist die Verwendung eines Extrakts aus Ginkgo-Biloba-Blättern zur Herstellung eines Mittels zur Behandlung von Schlafstörungen bei Patienten, deren Schlafarchitektur infolge einer verändenten Schlafregulation gegenüber derjenigen eines gesunden, jungen Probanden, im Schlaf-Elektroencephalogramm (Schlaf-EEG) durch eine Reduktion der Tiefschlafstadien, eine Verkürzung der Rapid-Eye-Movement-Latenz (REM-Latenz) und eine Erhöhung der Rapid-Eye-Movement-Dichte (REM-Dichte) gekennzeichnet ist.

Des weiteren betrifft die Erfindung die Verwendung von Extrakten aus Ginkgo-Biloba-Blättern als alleiniger Wirkstoff zur Herstellung eines Arzneimittels zur Behandlung von Schlafstörungen depressiver Patienten, insbesondere auch solcher depressiver Patienten, die sich in medikamentöser Antidepressiva-Behandlung befinden.

Die Erfindung betrifft weiterhin die Verwendung von Extrakten aus Ginkgo-Biloba-Blättern als alleiniger Wirkstoff zur Herstellung eines Arzneimittels zur Behandlung von Schlafstörungen von Patienten in höherem Alter. Es zeigt sich, dass die den Schlafstörungen depressiver Patienten ähnliche Schlafarchitektur von einem solchen Extrakt ebenfalls normalisierend positiv beeinflusst wird, und die Schlafeffizienz anheben kann.

Für die obigen Verwendungen wird vorzugsweise ein Extrakt unter Zuhilfenahme eines Aceton/Wasser Gemisches hergestellt, wie es in der Aufbereitungsmonographie für einen Trockenextrakt aus Ginkgo-Biloba-Blättern für den humanmedizinischen Bereich des deutschen Bundesgesundheitsamtes (Bundesanzeiger Nr. 133, 19.07.1994) vorgeschlagen wird.

### KURZE ERLÄUTERUNG DER FIGUREN

Die Erfindung soll nachfolgend anhand von Ausführungsbeispielen im Zusammenhang mit den Zeichnungen näher erläutert werden:
- Fig. 1: zeigt das mit einem Determinationsgerät zur Bestimmung von richtigen Reaktionen ermittelte mittlere Reaktionsverhalten der mit Trimipramin behandelten depressiven Patienten mit (gestrichelt, Quadrate) und ohne (ausgezogen, Kreise) Verabreichung des Ginkgo-Präparates;
- Fig. 2: zeigt eine Darstellung der Schlafarchitektur eines jungen, gesunden Menschen;
- Fig. 3: zeigt eine Darstellung der Schlafarchitektur eines depressiven Patienten;
- Fig. 4: zeigt die mittlere Tiefschlafdauer der mit Trimipramin behandelten depressiven Patienten in den verschiedenen Versuchsphasen (leere Säulen: ohne Ginkgo-Präparat, Kontrollgruppe; volle Balken: mit Ginkgo-Präparat, Querbalken mit X: Behandlungsdauer mit Ginkgo-Präparat);
- Fig. 5: zeigt den prozentualen Anteil von Tiefschlaf innerhalb der gesamten Schlafdauer derselben Probanden;
- Fig. 6: zeigt die mittlere Tiefschlafdauer in der ersten Nachthälfte derselben Probanden; und
- Fig. 7: zeigt eine Darstellung der Schlafarchitektur eines älteren Menschen.

### WEGE ZUR AUSFÜHRUNG DER ERFINDUNG

Die Wirkung von Trockenextrakten aus Blättern von Ginkgo-Biloba auf die kognitiven Fähigkeiten ist bekannt, und entsprechende Medikamente werden beispielsweise bei Hirnleistungsstörungen im Alter vom vaskulären, degenerativen und gemischten Typ in allen Stadien eingesetzt. So findet das Medikament Symfona® N der Anmelderin in der Schweiz seit längerer Zeit entsprechende Anwendung.

Hirnleistungsstörungen in Form von kognitiven Defiziten gehören auch bei depressiven Patienten zu den Kardinalsymptomen und werden als dementielles Syndrom der Depression bezeichnet. Ziel einer klinischen Studie war es nun entsprechend, bei depressiven Patienten zu untersuchen, ob eine zusätzliche Behandlung mit einem Ginkgo-Biloba-Präparat die therapeutische Wirksamkeit eines gängigen Thymoleptikums (in diesem Fall das antidepressiv Wirkende 5-[3-(Dimethylamino)-2-methylpropyl]-10,11-dihydro-5H-dibenz[b,f]-azepin, internat. Kurzbezeichnung Trimipramin) verbessert, insbesondere im Hinblick auf die gestörte Kognition.

An 16 Patienten mit Major Depression wurde untersucht, ob die zusätzliche Gabe von 240 mg Ginkgo-Biloba-Extrakt (Symfona® N) zur Verbesserung der Wirksamkeit von Trimipramin führt. 8 Patienten (5 Männer, 3 Frauen / Alter: 44,8+/-8.5 Jahre) wurden in einer offenen Monozenterstudie mit 200 mg Trimipramin für mindestens eine Woche behandelt. Anschliessend erhielten diese 8 Patienten für insgesamt 4 Wochen zusätzlich 240 mg Ginkgo-Biloba-Extrakt. In der letzten Woche der Studienphase wurde die zusätzliche Ginkgo-Gabe gestoppt und die gleichen 8 Patienten wieder mit einer Monotherapie von 200 mg Trimipramin behandelt. Die Ergebnisse dieser ersten Gruppe wurden mit 8 in Alter und Psychopathologie vergleichbaren Patienten als Kontrollgruppe (5 Männer, 3 Frauen / Alter: 49,5+/-9.7 Jahre) verglichen, die mit einer Monotherapie von 200 mg Trimipramin über den gesamten Studienzeitraum von 6 Wochen behandelt wurden. Die Messung der Zielvariablen zur Evaluation des Therapieeffekts aus den Bereichen kognitiv-psychomotorischer Leistungsbereich, Psychopathologie und Polysomnographie wurden mit Messungen zur Baseline, nach einer Woche (Evaluation des Effekts der akuten Ginkgo-Gabe), nach fünf Wochen (Evaluation des Effekts der längerfristigen Ginkgo-Gabe) und nach sechs Wochen (Evaluation des Absetzeffekts) durchgeführt.

Beispielhaft mögliche Formulierungen der verabreichten Ginkgo-Biloba-Präparate (Kapseln, Filmtabletten, Tropfen und Sirup) sind folgende (alle unter Verwendung eines Extraktes wie es in der Aufbereitungsmonographie für ein Trockenextrakt aus Ginkgo-Biloba-Blättern für den humanmedizinischen Bereich des deutschen Bundesgesundheitsamtes, Bundesanzeiger Nr. 133, 19.07.1994, vorgeschlagen wird):

### Beispiel 1: Kapseln

15 kg Ginkgo biloba Trockenextrakt werden mit Milchzucker, hochdispersem Siliciumdioxid und Magnesiumstearat gemischt und in 250 000 Hartgelatinekapseln mit einem Gehalt von je 60 mg Ginkgo biloba Trockenextrakt abgefüllt.

### Beispiel 2: Filmtabletten

15 kg Ginkgo biloba Trockenextrakt werden mit Maisstärke, hochdispersem Siliciumdioxid, Milchzucker, Zellulose, Dicalciumphosphat, Magnesiumstearat und Talk gemischt und zu Tabletten mit einem Gehalt von je 60 mg Ginkgo biloba Trockenextrakt gepresst. Diese Tabletten werden mit einem Film überzogen, der aus Polymeren wie Zellulosederivaten, Polyethylenglykol, Talk und Farbstoffen für den Lackfilm besteht.

### Beispiel 3: Tropfen

9 kg Ginkgo biloba Trockenextrakt werden in Propylenglykol gelöst. Diese Lösung wird mit einer wässrigen Lösung vermischt, die verschiedene Süssstoffe, Aromatika, Geschmackskorrigentien und Puffersalze enthält.

### Beispiel 4: Sirup

0,9 kg Ginkgo biloba Trockenextrakt werden in Propylenglykol gelöst und mit einer wässrigen Lösung vermischt, die aus Glycerol, verschiedenen Süssstoffen, Aromenstoffen und Puffersalzen besteht.

In den vorstehenden Beispielen enthalten die Kapseln und die Filmtabletten jeweils 60 mg Ginkgo biloba Trockenextrakt, der auf mindestens 25 % Flavonglykoside und mindestens 6 % Terpenlaktone standardisiert ist, das heisst auf mindestens 15 mg Flavonglykoside und 3,6 mg Terpenlaktone je Darreichungsform. Die Tropfen und der Sirup enthalten 80 mg bzw. 6 mg des Trockenextrakts pro ml Lösung. Anwendung finden beispielsweise die von der Anmelderin unter dem Namen Symfona®-N in der Schweiz vertriebenen Produkte, welche Ginkgo biloba Extrakt (Li 1370) enthalten. Die Kapseln Symfona®-N beispielsweise enthalten 60mg Extrakt/Kapsel.

Eine Verabreichung von zweimal täglich je einer Kapsel bzw. Filmtablette, von 1 ml Tropfen oder 10 ml Sirup entspricht einer mittleren Tagesdosis von 120 mg Trockenextrakt. Für die hier beschriebenen Anwendungen sind aber mittlere Tagesdosen in einem Bereich, welcher 60 - 300mg Trockenextrakt entspricht, sinnvoll einsetzbar.

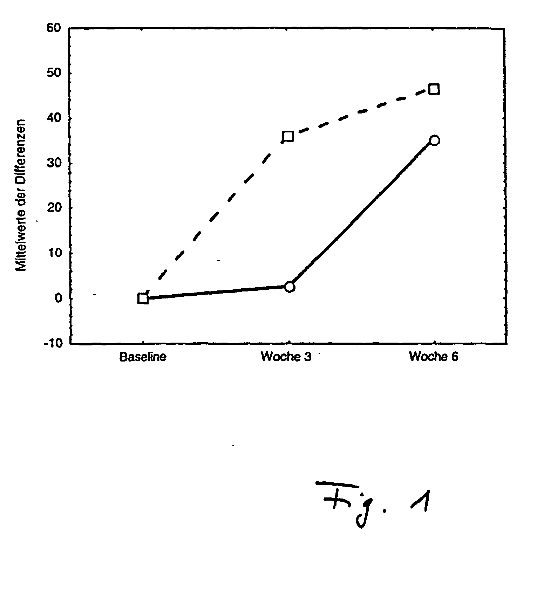

Wie aufgrund der bekannten Wirksamkeit von Ginkgo-Biloba auf die Kognition erwartet werden durfte, zeigte sich in der ersten Ginkgo-Gruppe eine signifikante Leistungssteigerung und erhöhte Stressresistenz in komplexen Situationen in denen eine Vielzahl unterschiedlicher Reize unter Zeitdruck adäquat verarbeitet werden mussten. Sowohl die tendenzielle Reaktionszeitverkürzung im Tracking-Gerät als auch die deskriptive Abnahme der verspätet richtigen Reaktionen und die Zunahme der richtigen Reaktionen deuten, wie in Figur 1 dargestellt (ausgezogene Linie: Behandlung mit Trimipramin, Kontrollgruppe; gestrichelte Linie: Behandlung mit Trimipramin und Ginkgo-Präparat; zunehmende y-Achse: zunehmende richtige Reaktionen), auf eine erhöhte Aufmerksamkeit und Reagibilität unter Ginkgo hin. Dieser Befund wird durch eine Reihe von weiteren Tests zur Vigilanz unterstützt.

Überraschend und völlig neu im Zusammenhang mit der Verabreichung von Symfona® N waren aber die Resultate der polysomnographischen Untersuchungen.

Der Schlaf wird heute zunehmend wissenschaftlich in sogenannten Schlaflabors untersucht, nicht zuletzt um den Ursachen von Schlafstörungen auf den Grund zu kommen. In Schlaflabors werden eine Reihe von sommnographischen Kenngrössen erfasst: Mit am Kopf anliegenden Elektroden werden die im Schlaf auftretenden Hirnströme gemessen. Der zeitliche Verlauf des resultierenden Schlaf-Elektro-Encephalogramms (Schlaf-EEG) weist für verschiedene Schlafphasen ein unterschiedliches charakteristisches Verhalten auf. Im Bereich der Augenmuskeln angebrachte Elektroden erlauben die Messung der Augenbewegung während des Schlafes. Auch das daraus resultierende Elektrookulogramm (Schlaf-EOG) weist einen für bestimmte Schlafphasen charakteristischen zeitlichen Verlauf auf. Insbesondere die als Rapid-Eye-Movement (REM) bekannte Phase kann mittels EOGs ermittelt werden. Ausserdem kann mittels geeigneter Untersuchung des Blutes der darin enthaltene Spiegel bestimmter Hormone und Neuropeptide bestimmt werden. Als für den Schlafverlauf in diesem Zusammenhang relevant und charakteristisch haben sich vor allem ein Wachstumshormon (Growth hormone releasing hormone, GHRH) und ein Kortikotropin freisetzendes Hormon (Corticotropin-releasing hormone, CRH) herausgestellt. Letzteres löst mehr oder weniger direkt die Ausschüttung von Cortisol aus, weshalb auch dieses als entsprechende Kenngrösse dienen kann.

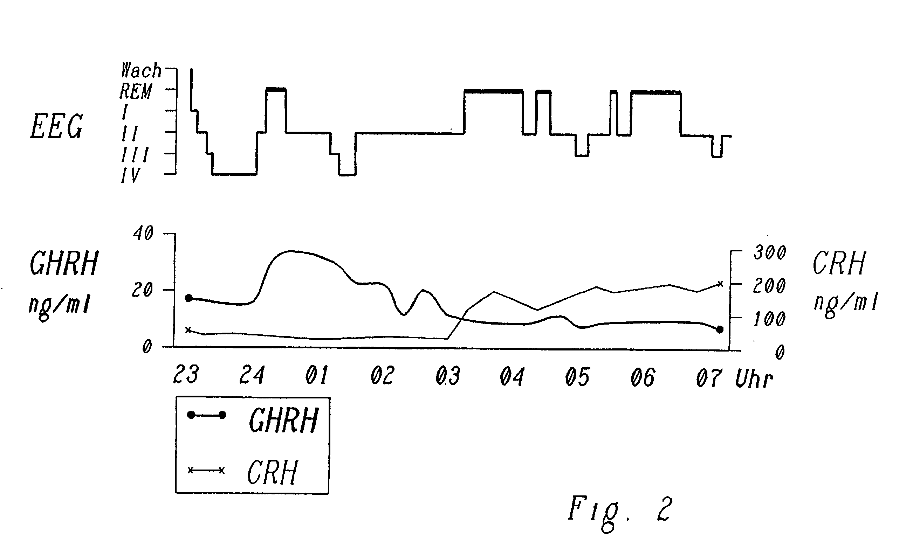

Figur 2 zeigt einen typischen, in einem Schlaflabor bestimmten Verlauf der oben beschriebenen Kenngrössen für einen gesunden, jungen Probanden. In dieser sogenannten Schlafarchitektur wird der mittels EEG und EOG bestimmte Schafzustand in 5 Phasen eingeteilt. Zunächst die REM-Phase, welche hier hauptsächlich in der zweiten Nachthälfte auftritt und dort ziemlich lange anhalten kann. Anschliessend folgen die vier Non-REM-Phasen, welche einen gewissermassen graduell "tieferen" Schaf bezeichnen, und deren Stufe IV für den sogenannten Tiefschlaf steht. Tiefschlaf tritt bei einem jungen Patienten hauptsächlich in der ersten Nachthälfte auf, und dauert dort bis zu einer Stunde. Daneben sind in Figur 1 die beiden obenerwähnten Botenstoffe, das Wachstumshormon (GHRH) und das Cortisol als Funktion der Zeit aufgetragen. Man beobachtet bei einem jungen, gesunden Patienten zunächst eine Zunahme des Spiegels von GHRH im Blut auf bis zu 40 ng/ml, welcher dann im wesentlichen stetig abklingt. Gegenläufig dazu nimmt in der zweiten Nachthälfte das Cortisol kontinuierlich zu bis auf Werte von 200 ng/ml.

Schlafstörungen können sich nun auf die verschiedenen obenerwähnten Observablen unterschiedlich auswirken, und das hormonelle Gleichgewicht verändert sich dabei in die Schlafarchitektur beeinflussender Weise. Es ist eine Fülle von Mitteln zur Behandlung von Schlafstörungen bekannt, meist weisen diese aber eine Reihe von z.T. bei chronischer Einnahme gravierenden Nebenwirkungen wie Nachlassen der kognitiven Eigenschaften, Schläfrigkeit während des Tages u.s.w. auf.

Auch bei depressiven Patienten treten als weiteres Kardinalsymptom bekanntermassen Schlafstörungen auf. Depressive Patienten zeigen eine oder mehrere der folgenden Auffälligkeiten im Schlaf-EEG:
◆ Störungen der Schlafkontinuität mit verlängerter Einschlaflatenz, häufiges nächtliches Erwachen und Früherwachen.
◆ Störungen der Schlafarchitektur mit Verschiebung von REM-Schlaf in die erste Nachthälfte, Verkürzung der REM-Latenz und Erhöhung der REM-Dichte.
◆ Verringerung des Tiefschlafes, insbesondere Stadium 4.

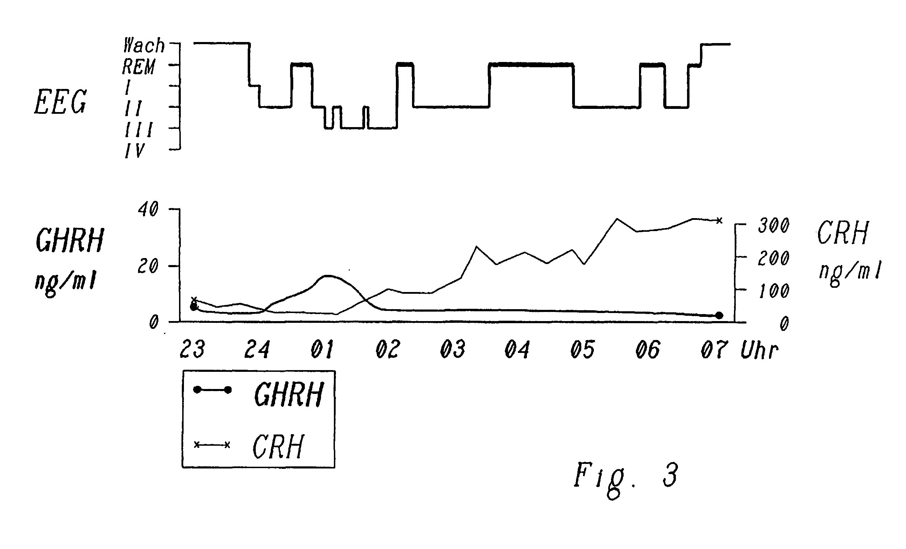

Eine entsprechende Schlafarchitektur ist in Figur 3 dargestellt. Man erkennt neben den obenerwähnten Effekten eine wesentlich reduzierte Ausschüttung von GHRH und eine etwas verstärkte Ausschüttung von Cortisol.

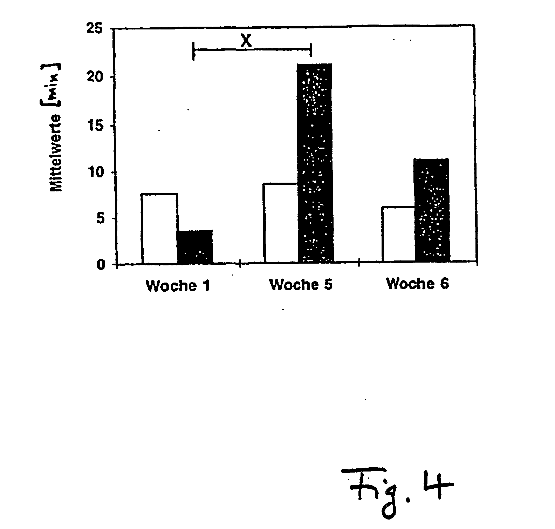

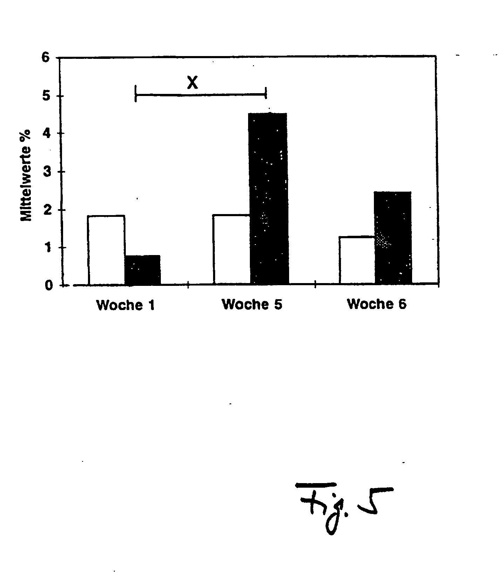

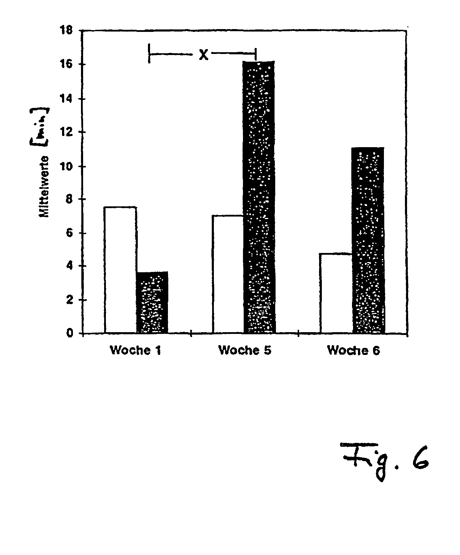

Als wesentlichster Befund ergibt sich, wie in den Figuren 4 bis 6 erkennbar ist, während und nach vierwöchiger Zusatztherapie mit Ginkgo-Biloba-Präparaten eine signifikante Zunahme des Tiefschlafes, insbesondere von Stadium IV. Figur 4 zeigt den Mittelwert der gesamten Tiefschlafdauer (Stadium IV) in Minuten während der ganzen Nacht. Die leeren Balken bezeichnen die Kontrollgruppe, die ausgefüllten Balken die Gruppe mit Ginkgo-Zusatzbehandlung. Der mit "X" bezeichnete Horizontalbalken stellt die Zeit der Verabreichung von Ginkgo für letztere Gruppe an. Figur 5 zeigt analog den mittleren prozentualen Anteil von Tiefschlaf (Stadium IV) bezogen auf die gesamte Schlafzeit. Figur 6 zeigt ebenfalls analog, die gesamte Tiefschlafdauer (Stadium IV) in Minuten innerhalb der ersten Hälfte der Nacht.

Der Effekt wird durch die Beobachtung der Tiefschlafabnahme nach Absetzen der Ginkgo-Zusatztherapie untermauert. Darüber hinaus sind weitere Effekte auf den Schlaf zu beobachten: Bereits nach kurzfristiger Ginkgo-Gabe kommt es zu einer signifikanten Zunahme der Schlafeffizienz sowie zu einer Abnahme der Frequenz der Aufwachphasen. Des weiteren zeigte sich eine Verlängerung der REM-Latenz und eine Reduktion der REM-Dichte in der gesamten Nacht mit einer Zunahme des Anteils an Non-REM-Schlaf (Stadien 1 bis IV). Auch bei diesen Befunden besteht nach Absetzen der Ginkgo-Gabe ein klarer Hinweis auf eine inverse Regulation des REM-Schlafes, indem sich ein Vorrücken des REM-Schlafes mit verkürzter REM-Latenz nachweisen lässt.

Abschliessend lässt sich sagen, dass die Verabreichung des Ginkgo biloba Präparates bei depressiven Patienten mit typischen Schlafstörungen zu einer normalisierung der Schlafarchitektur und damit zu einer raschen Besserung der Schlafstörung führt.

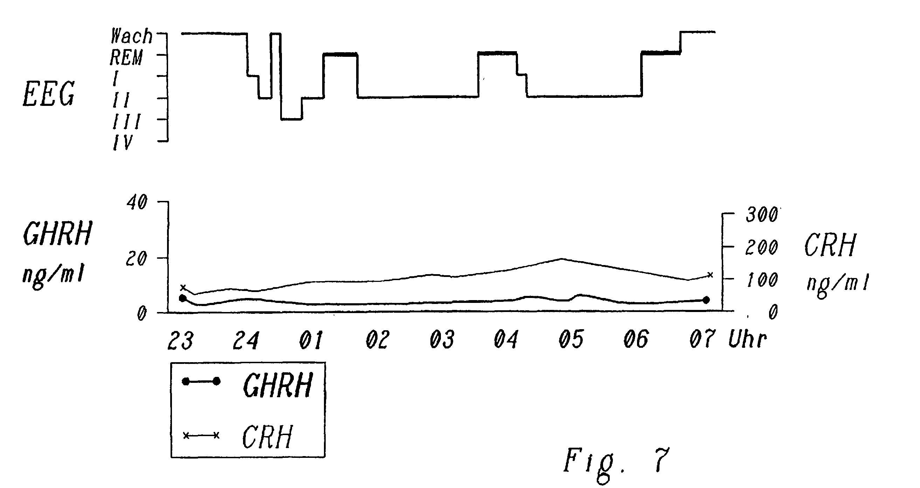

Der Schlaf des Menschen ist charakterisiert durch den zyklischen Wechsel von Non-REM und REM-Perioden im Schlaf-EEG und durch bestimmte Muster der nächtlichen Hormonsekretion. Im Verlauf des normalen Alterns und in der Depression treten gleichsinnige Veränderungen der schlafendokrinologischen Aktivität auf unter anderem eine Verminderung von Wachstumhormonsekretion (GHRH) und Tiefschlaf. Es ist heute, wie schon oben erwähnt, erwiesen, dass Neuropeptide eine Schlüsselrolle in der Schlafregulation einnehmen. So ist Wachstumshormon freisetzendes Hormon (GHRH) der gemeinsame Stimulus von Tiefschlaf, während Kortikotropin freisetzendes Hormon (CRH) sein Gegenspieler ist. Ein Ungleichgewicht zwischen diesen beiden Peptiden zugunsten von CRH dürfte wesentlich zur Entstehung von Schlafstörungen in der Depression und im zunehmenden Alter beitragen. Das in Figur 7 dargestellte Schlafprofil entpricht dem eines Menschen in zunehmendem Alter, d.h. oberhalb von 60 Jahren (natürlich ist das Altern sehr individuell, und entsprechend auch die Definition des "zunehmenden Alters"). Klar erkennbar ist wiederum erhöhte Schlaflatenz, wenig Tiefschlaf (Stadium IV) und frühes Erwachen (senile Bettflucht). Man erkennt, dass kaum GHRH ausgeschüttet wird, und nur ein schwacher Anstieg der Cortisol-Ausschüttung eintritt.

Aufgrund der geschilderten Ähnlichkeit der pathologischen Schlafarchitektur bei Schlafstörungen alternder Menschen und derer depressiver Menschen ist es leicht ersichtlich, dass eine Ginkgo biloba Verabreichung auch bei Schlafproblemen alternder Menschen die vorliegenden überraschenden positiven Effekte zeigt und die Schlafarchitektur normalisieren kann.

## Patentansprüche

1. Verwendung eines Extraktes aus Ginkgo-Biloba-Blättern zur Herstellung eines Mittels zur Behandlung von Schlafstörungen bei Patienten, deren Schlafarchitektur infolge einer veränderten Schlafregulation gegenüber derjenigen eines gesunden, jungen Probanden im Schlaf-Elektroencephalogramm (Schlaf-EEG) durch eine Reduktion der Tiefschlafphasen, eine Verkürzung der Rapid-Eye-Movement-Latenz (REM-Latenz) und eine Erhöhung der Rapid-Eye-Movement-Dichte (REM-Dichte) gekennnzeichnet ist.

2. Verwendung nach Anspruch 1, dadurch gekenzeichnet, dass die Schlafregulation durch Auftreten eines Ungleichgewichts zwischen einem Wachstumshormon freisetzenden Hormon (GHRH) und einem Kortikotropin freisetzenden Hormon (CRH) gegenüber dem Verhältnis dieser Neuropeptide bei einem gesunden, jungen Probanden verändert ist.

3. Verwendung nach Anspruch 1 oder 2, **dadurch gekennzeichnet, dass** die Schlafregulation durch eine verminderte Ausschüttung von einem Wachstumshormon freisetzenden Hormon (GHRH) gegenüber derjenigen bei einem gesunden, jungen Probanden verändert ist.

4. Verwendung nach einem der Ansprüche 1 - 3, **dadurch gekennzeichnet, dass** die Schlafregulation gegenüber derjenigen eines gesunden, jungen Probanden entsprechend derjenigen bei depressiven Patienten verändert ist.

5. Verwendung nach Anspruch 1 -3, **dadurch gekennzeichnet, dass** die Schlafregulation gegenüber derjenigen eines gesunden, jungen Probanden entsprechend derjenigen bei Patienten in höherem Alter verändert ist.

6. Verwendung eines Extraktes aus Ginkgo-Biloba-Blättern als alleiniger Wirkstoff zur Herstellung eines Arzneimittels zur Behandlung von Schlafstörungen depressiver Patienten.

7. Verwendung eines Extraktes aus Ginkgo-Biloba-Blättern als alleiniger Wirkstoff zur Herstellung eines Arzneimittels zur Behandlung von Schlafstörungen sich in medikamentöser Antidepressiva-Behandlung befindlicher depressiver Patienten.

8. Verwendung eines Extraktes aus Ginkgo-Biloba-Blättem als alleiniger Wirkstoff zur Herstellung eines Arzneimittels zur Behandlung von Schlafstörungen von Patienten in höherem Alter.

9. Verwendung nach einem der Ansprüche 1 - 8, **dadurch gekennzeichnet, dass** der Extrakt ein Trockenextrakt ist.

## Claims

1. A use of an extract of ginkgo biloba leaves for preparation of an agent for treatment of sleep disorders in patients whose sleep architecture is **characterized by** a reduction in deep-sleep phases, shortening of rapid eye movement latency (REM latency) and an increase in rapid eye movement density (REM density) due to an altered sleep regulation in comparison with that of a healthy young volunteer in the sleep electroencephalogram (sleep EEG).

2. The use according to Claim 1, **characterized in that** the sleep regulation is altered due to the development of an imbalance between a growth hormone-releasing hormone (GHRH) and a corticotropin-releasing hormone (CRH) in comparison with the ratio of these neuropeptides in a healthy, young volunteer.

3. The use according to Claim 1 or 2, **characterized in that** the sleep regulation is altered in comparison with that in a healthy, young volunteer due to a decreased secretion of a growth hormone-releasing hormone (GHRH).

4. The use according to one of Claims 1 through 3, **characterized in that** the sleep regulation is altered like that in depressive patients in comparison with that in a healthy, young volunteer.

5. The use according to Claims 1 through 3, **characterized in that** sleep regulation is altered like that in patients of advanced age in comparison with that in a healthy, young volunteer.

6. The use of an extract of ginkgo biloba leaves as the exclusive active ingredient for preparing a medication for treatment of sleep disorders in depressive patients.

7. The use of an extract of ginkgo biloba leaves as the exclusive active ingredient for preparing a medication for treatment of sleep disorders in depressive patients under treatment with antidepressant medication.

8. The use of an extract of ginkgo biloba leaves as the exclusive active ingredient for preparing a medication for treatment of sleep disorders in patients of advanced age.

9. The use according to one of Claims 1 through 8, **characterized in that** the extract is a dry extract.

## Revendications

1. Utilisation d'un extrait de feuilles de ginkgo biloba pour la fabrication d'un moyen pour le traitement de troubles du sommeil chez des patients dont l'architecture du sommeil à la suite d'une régulation perturbée du sommeil par rapport à des volontaires sains et jeunes est caractérisée dans l'électroencéphalogramme du sommeil (EEC du sommeil) par une réduction des phases de sommeil profond, une réduction de la latence Rapid-Eye-Movement (sommeil paradoxal) (latence REM) et une augmentation de la densité du sommeil paradoxal (Densité REM).

2. Utilisation selon la revendication 1, **caractérisée en ce que** la régulation du sommeil est modifiée par la survenance d'un déséquilibre entre une hormone libérant l'hormone de croissance (GHRH) et une hormone libérant la corticotropine Vis-à-vis du rapport de ces neuropeptides chez un volontaire jeune et sain.

3. Utilisation selon la revendication 1 ou 2, **caractérisée en ce que** la régulation du sommeil est modifiée par rapport à celle d'un volontaire jeune et sain.

4. Utilisation selon l'une des revendications 1 à 3, **caractérisée en ce que** la régulation du sommeil est modifiée par un dégagement diminué d'une hormone libérant l'hormone de croissance (GHRH) par rapport à celle d'un volontaire jeune et sain en fonction de celle des patients dépressifs.

5. Utilisation selon l'une des revendications 1 à 3, **caractérisée en ce que** la régulation du sommeil est modifiée par rapport à celle d'un volontaire jeune et sain en fonction de celle des patients plus âgés.

6. Utilisation d'un extrait de feuilles de ginkgo biloba en tant que substance active exclusive pour la fabrication d'un médicament en vue du traitement de troubles du sommeil chez des patients dépressifs.

7. Utilisation d'un extrait de feuilles de ginkgo biloba en tant que substance active exclusive pour la fabrication d'un médicament en vue du traitement de troubles du sommeil chez des patients dépressifs prenant des médicaments anti-dépresseurs.

8. Utilisation d'un extrait de feuilles de ginkgo biloba en tant que substance active exclusive pour la fabrication d'un médicament en vue du traitement de troubles du sommeil chez des patients âgés.

9. Utilisation selon l'une des revendications 1 à 8, **caractérisée en ce que** l'extrait est un extrait sec.
